# EUROPÄISCHE PATENTSCHRIFT

(11) **EP 2 779 894 B1**
(45) Veröffentlichungstag und Bekanntmachung des Hinweises auf die Patenterteilung: **18.10.2017**
(21) Anmeldenummer: 12798136.3
(22) Anmeldetag: 17.10.2012
(51) Int. Cl.: A61B 5/053, G01G 19/44

(54) **VERFAHREN UND VORRICHTUNG ZUR ERMITTLUNG DES KÖRPERGEWICHTES EINER PERSON**
METHOD AND DEVICE FOR DETERMINING THE BODY WEIGHT OF A PERSON
PROCÉDÉ ET DISPOSITIF DE DÉTERMINATION DU POIDS CORPOREL D'UNE PERSONNE

(30) Priorität: 14.11.2011 DE 102011118998
(43) Veröffentlichungstag der Anmeldung: 24.09.2014
(73) Patentinhaber: seca ag, 4153 Reinach BL 1 (CH)
(72) Erfinder: VOGEL, Frederik, 22301 Hamburg (DE)
(74) Vertreter: Patentanwälte Klickow & Partner Partnerschaftsgesellschaft mbB
(86) Internationale Anmeldenummer: PCT/DE2012/001024
(87) Internationale Veröffentlichungsnummer: WO 2013/071903

(56) Entgegenhaltungen:
- US-A1- 2006 030 783
- US-A1- 2010 098 310

## Beschreibung

Die Erfindung betrifft ein Verfahren zur Ermittlung des Körpergewichtes einer Person, bei dem messtechnisch mindestens ein für eine Bioimpedanzmessung typischer Parameter erfasst und zu einer Auswertungseinrichtung übermittelt wird.

Die Erfindung betrifft darüber hinaus eine Vorrichtung zur Ermittlung des Körpergewichtes einer Person, die mindestens einen Sensor zur Erfassung mindestens eines für eine Bioimpedanzmessung typischen Parameters aufweist und bei der der Sensor mit einer Auswertungseinrichtung gekoppelt ist.

Die Gewichtsbestimmung bei einer überwiegenden Anzahl von Personen kann in einfacher Weise dadurch erfolgen, dass sich diese Personen auf eine Waage stellen und das entsprechende Körpergewicht dann angezeigt wird. Als problematisch erweist sich die Gewichtsbestimmung bei bettlägerigen Patienten und/oder Pflegebedürftigen. Gerade bei diesen Personen ist aber die Bestimmung des Körpergewichtes von besonderer Bedeutung, um Rückschlüsse auf den Ernährungszustand gewinnen zu können. Häufig tritt bei derartigen Personen das Problem auf, dass diese über- oder unterernährt werden, was zu unerwünschten Gewichtszunahmen oder -abnahmen führt. Gleichfalls kann das Problem auftreten, dass fehlende Bewegung und Stimulation zu einem Abbau von Muskeln führt.

Um das Körpergewicht derartiger Personen festzustellen ist es häufig erforderlich, die Patienten aus dem Bett herauszuheben und/oder zu bewegen. Soweit als möglich werden die Personen dann auf eine Waage gestellt oder im Sitzen gewogen. Als noch aufwendiger gestaltet sich die Bestimmung des Körpergewichtes durch das Wiegen des Bettes gemeinsam mit dem Patienten und eine korrespondierende Ermittlung nur des Gewichtes des Bettes. Auch ein derartiger Ablauf ist mit erheblichen Schwierigkeiten verbunden, da gerade bei Pflegebedürftigen die Betten oft nicht von allen Seiten gut zugänglich sind. Darüber hinaus sind die Patienten häufig an Mess- oder Versorgungsgeräte angeschlossen, deren Eigengewicht ebenfalls zu berücksichtigen ist.

Die bisherigen Arbeitsabläufe bei der Ermittlung des Körpergewichtes von bettlägerigen Patienten können somit arbeitsaufwendig, ungenau und für den Patienten zumindest mit Unannehmlichkeiten und häufig auch mit Schmerzen verbunden sein.

Aus der US 2006/030783 A1 ist bereits ein Verfahren zur Ermittlung des Körpergewichtes einer Person bekannt. Es wird messtechnisch mindestens ein für eine Bioimpedanzmessung typischer Parameter erfasst. Der erfasste Parameter wird zu einer Auswertungseinrichtung übermittelt. Die Berechnung des Körpergewichtes erfolgt ausschließlich unter Berücksichtigung von unter Verwendung von Sensoren gemessenen Werten.

Gemäß der US 2010/098310 A1 erfolgt eine Schätzung eines Gewichtes eines Patienten. Die Schätzung erfolgt auf Basis eines Röntgenbildes.

Aufgabe der vorliegenden Erfindung ist es daher, ein Verfahren der einleitend genannten Art derart zu verbessern, dass die Gewichtsbestimmung bei liegenden oder sitzenden Patienten unterstützt wird.

Diese Aufgabe wird erfindungsgemäß dadurch gelöst, dass die Auswertungseinrichtung den Messwert gemeinsam mit einzelnen oder mehreren weiteren individuellen Daten der Person verarbeitet und unter Berücksichtigung von statistischen Daten zu anderen Personen einen möglichst genauen Schätzwert für das Gewicht der Person ermittelt.

Weitere Aufgabe der vorliegenden Erfindung ist es, eine Vorrichtung der einleitend genannten Art derart zu konstruieren, dass die Bestimmung des Gewichtes von liegenden Personen unterstützt wird.

Diese Aufgabe wird erfindungsgemäß dadurch gelöst, dass die Auswertungseinrichtung sowohl einen Individualspeicher für individuelle Daten der Person als auch einen Referenzspeicher für statistische Daten von anderen Personen aufweist und dass die Auswertungseinheit zur Verknüpfung des Messparameters sowohl mit Inhalten des Individualspeichers als auch mit Inhalten des Referenzspeichers ausgebildet ist.

Das erfindungsgemäße Verfahren und die Vorrichtung ermöglichen es, den Ernährungszustand eines Patienten zu ermitteln, ohne das tatsächliche Gewicht des Patienten exakt zu kennen. Es ist somit insbesondere möglich, Zunahmen oder Abnahmen des Körpergewichtes darzustellen. Dies kann mit oder ohne eine Verlaufsmessung erfolgen. Insbesondere ist es möglich, das Gewicht des Patienten zu ermitteln, ohne diesen bewegen zu müssen.

Eine Ermittlung des Messwertes kann bevorzugt unter Verwendung eines sogenannten Body-Composition-Analysers (BCA) erfolgen. Eine derartige Vorrichtung ist beispielsweise dafür geeignet, Gewichtsanteile von Muskeln, Fett, Wasser und Knochen innerhalb des menschlichen Körpers zu bestimmen. Typischerweise werden hierbei weitere Kennwerte der betreffenden Person als Einflussparameter berücksichtigt, beispielsweise das Alter, das Geschlecht sowie die Ethnie. Das Gewicht der betreffenden Person wird dabei üblicherweise durch einen Wiegevorgang bestimmt. Bei einer üblichen Verwendung eines derartigen BCA-Gerätes wird das Gewicht der Person somit als Einflussparameter eines Auswertungsalgorhythmus berücksichtigt. Tatsächliche Messwerte des BCA-Gerätes sind Impedanzwerte des Körpers zwischen vorbestimmten Messpunkten. Die Messpunkte befinden sich typischerweise im Bereich der Hände oder der Füße der betreffenden Person.

Erfindungsgemäß wird nun die funktionelle Abhängigkeit der Werte der BCA-Gerät ermittelten Parameter vom Gewicht der Person dazu genützt, mathematisch gesehen durch eine Auflösung der betreffenden funktionellen Abhängigkeit nach dem Gewicht dieses Gewicht zu bestimmen. Durch eine zusätzliche Berücksichtigung von statistischen Daten von Vergleichspersonen lässt sich die Genauigkeit der Ermittlung des Gewichtes verbessern.

Eine erhöhte Genauigkeit bei der Ermittlung der Schätzwerte für das Gewicht der Person lässt sich dadurch erreichen, dass die Auswertungseinrichtung zusätzlich mindestens einen zeitlich zurückliegenden Messwert berücksichtigt.

Insbesondere ist darin gedacht, dass eine Verlaufsermittlung durchgeführt wird.

In den Zeichnungen sind Ausführungsbeispiele der Erfindung schematisch dargestellt. Es zeigen:
- Fig. 1: Eine schematische Darstellung eines Blockschaltbildes zur Veranschaulichung der Grundstruktur der verwendeten Vorrichtung,
- Fig. 2: eine beispielhafte Darstellung eines Verlaufes des Körpergewichtes aufgetragen über die Zeit und

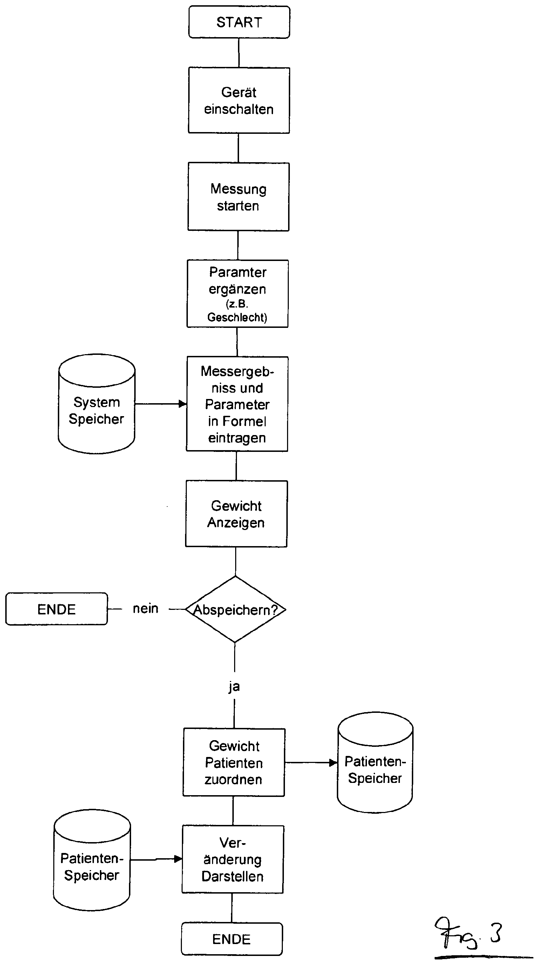

- Fig. 3: ein Ablaufdiagramm zur Veranschaulichung der Durchführung des erfindungsgemäßen Verfahrens.

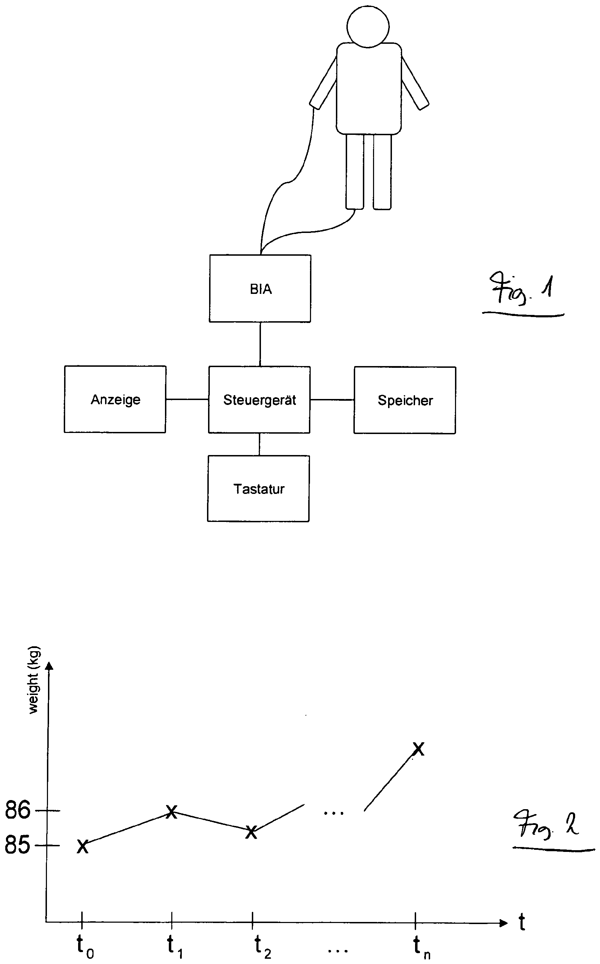

Fig. 1 zeigt schematisch eine Person (1), die in einem liegenden Zustand über Messkabel (2, 3) an eine Messeinrichtung (4) angeschlossen ist. Die Messeinrichtung (4) ist mit einem Steuergerät (5) verbunden. Das Steuergerät (5) stellt typischerweise eine Auswertungseinrichtung bereit.

Zur Ermöglichung von Bedieneingaben kann das Steuergerät (5) mit einer Tastatur (6) gekoppelt sein. Alternativ können auch andere Eingabeeinrichtungen verwendet werden.

Darüber hinaus ist das Steuergerät (5) mit einer Anzeige (7) und einem Speicher (8) gekoppelt.

Gemäß einem typischen Verfahrensablauf wird die betreffende Person zu einem ersten Zeitpunkt (T0) unter Verwendung der erfindungsgemäßen Vorrichtung vermessen. Dieser Wert wird in der Vorrichtung abgespeichert, bevorzugt unter Zuordnung einer eindeutigen Identifikationsnummer des betreffenden Patienten. Sollte aus zeitlich zurückliegenden Messungen das tatsächliche Gewicht des Patienten zu einem zurückliegenden Zeitpunkt (TP) bekannt sein, so kann auch dieses zuletzt bekannte tatsächliche Gewicht mit berücksichtigt werden.

Gemäß einer bevorzugten Verfahrensdurchführung wird bei Folgemessungen zu Zeitpunkten (TX) gegen die Werte des Zeitpunktes (T0) gemessen. Dies unterstützt die Durchführung einer Verlaufsmessung sowie Verlaufsdarstellung. Dies ermöglicht es für einen Betreuer des Patienten zu erkennen, ob sich der Ernährungszustand des Patienten verändert. Insbesondere ist dies somit möglich, ohne dass der tatsächliche exakte Wert des Körpergewichtes bekannt ist.

Zur Dokumentation der jeweiligen Messergebnisse ist es möglich, die Vorrichtung mit einem Drucker auszustatten. Ebenfalls ist die Durchführung von Dauermessungen möglich. Eine gerätetechnische Ausführung kann portabel oder stationär sein.

## Patentansprüche

1. Verfahren zur Ermittlung des Körpergewichtes einer Person, bei dem messtechnisch mindestens ein für eine Bioimpedanzmessung typischer Parameter erfasst und zu einer Auswertungseinrichtung übermittelt wird, **dadurch gekennzeichnet, dass** die Auswertungseinrichtung den Messwert gemeinsam mit einzelnen oder mehreren weiteren individuellen Daten der Person verarbeitet und unter Berücksichtigung von statistischen Daten zu anderen Personen einen Schätzwert für das Gewicht des Patienten ermittelt.

2. Verfahren nach Anspruch 1, **dadurch gekennzeichnet, dass** die Auswertungseinrichtung zusätzlich mindestens einen zeitlich zurückliegenden Messwert berücksichtigt.

3. Verfahren nach Anspruch 1 oder 2, **dadurch gekennzeichnet, dass** eine Verlaufsermittlung durchgeführt wird.

4. Vorrichtung zur Ermittlung des Körpergewichtes einer Person, die mindestens einen Sensor zur Erfassung mindestens eines für eine Bioimpedanzmessung typischen Parameters aufweist und bei der der Sensor mit einer Auswertungseinrichtung gekoppelt ist, wobei die Auswertungseinrichtung einen Individualspeicher für individuelle Daten der Person enthält, **dadurch gekennzeichnet, dass** die Auswertungseinrichtung auch einen Referenzspeicher für statistische Daten zu anderen Personen aufweist und dass die Auswertungseinrichtung zur Verknüpfung des Messparameters sowohl mit Inhalten des Individualspeichers als auch mit Inhalten des Referenzspeichers ausgebildet ist.

## Claims

1. Method for determining the body weight of a person, whereby at least one parameter typical of a biological impedance measurement is detected by measuring means and transmitted to an evaluation unit, **characterised in that** the evaluation unit processes the measurement value together with individual or a number of other individual data items pertaining to the person and determines an estimated value for the weight of the patient taking account of statistical data pertaining to other persons.

2. Method as claimed in claim 1, **characterised in that** the evaluation unit additionally takes into account at least one measurement value from an earlier point in time.

3. Method as claimed in claim 1 or 2, **characterised in that** a graph of measurements is plotted.

4. Device for determining the body weight of a person, comprising at least one sensor for detecting at least one parameter typical of a biological impedance measurement and the sensor is coupled with an evaluation unit, which evaluation unit contains an individual memory for individual data pertaining to the person, **characterised in that** the evaluation unit further comprises a reference memory for statistical data pertaining to other persons, and the evaluation unit is configured to correlate the measurement parameter with both the contents of the individual memory and the contents of the reference memory.

## Revendications

1. Procédé de détermination du poids corporel d'une personne, dans lequel au moins un paramètre typique pour une mesure de bio impédance est saisi selon la technique de mesure et est transmis à un système d'évaluation,
**caractérisé en ce que** le système d'évaluation traite la valeur mesurée en commun avec certaines données individuelles ou avec plusieurs autres données individuelles de la personnes et détermine une valeur estimative concernant le poids du patient, en tenant compte de données statistiques relatives à d'autres personnes.

2. Procédé selon la revendication 1,
**caractérisé en ce que** le système d'évaluation tient de plus compte d'au moins une valeur mesurée plus ancienne.

3. Procédé selon revendication 1 ou 2,
**caractérisé en ce que** l'on exécuté une détermination du déroulement.

4. Dispositif destiné à la détermination du poids corporel d'une personne, lequel est doté d'au moins un détecteur, qui est destiné à saisir au moins un paramètre typique pour une mesure de bio impédance, et dans lequel le détecteur est couplé avec un système d'évaluation, sa-chant que le système d'évaluation est également doté d'une mémoire de référence contenant des données statistiques d'autres personnes et que le système d'évaluation est conçu pour associer le paramètre aussi bien aux contenus de la mémoire individuelle qu'aux contenus de la mémoire de référence.
